Europäisches Patentamt

European Patent Office

Office européen des brevets

(19)

(11) Veröffentlichungsnummer: **0 078 425**
**B1**

(12)                     # EUROPÄISCHE PATENTSCHRIFT

(45) Veröffentlichungstag der Patentschrift:
13.08.86

(21) Anmeldenummer: **82109500.7**

(22) Anmeldetag: **14.10.82**

(51) Int. Cl.⁴: **A 61 B 6/14**

(54) **Hilfsmittel für die Durchführung von Röntgenuntersuchungen an Zähnen.**

(30) Priorität: **04.11.81 SE 8106534**

(43) Veröffentlichungstag der Anmeldung:
**11.05.83 Patentblatt 83/19**

(45) Bekanntmachung des Hinweises auf die Patenterteilung:
**13.08.86 Patentblatt 86/33**

(84) Benannte Vertragsstaaten:
**AT BE CH DE FR GB IT LI NL SE**

(56) Entgegenhaltungen:
**DE-A-1 932 743**
**US-A-3 092 721**
**US-A-3 304 422**

(73) Patentinhaber: **Trollhätteplast Försäljning AB, Box
55, S-461 22 Tröllhättan (SE)**

(72) Erfinder: **Welander, Ulf, Prof., Sofielundsvägen 16,
S-902 37 Umea (SE)**

(74) Vertreter: **Wendl, Christl, Schlossplatz 1 Postfach
1329, D-7758 Meersburg (DE)**

EP 0 078 425 B1

## Beschreibung

Die Erfindung bezieht sich auf ein Hilfsmittel für die Durchführung von Röntgenuntersuchungen an Zähnen, mit einem Filmhalter L-förmigen Querschnitts zur Aufnahme eines Röntgenfilms in zwei alternativen Ebenen.

Ein derartiges Hilfsmittel, bei dem der Filmhaltert mit einem Verlängerungsstück schwenkbar verbunden ist, ist bereits aus der US-A-3 092 721 bekannt (vgl. Fig. 9). Ferner ist aus dieser Druckschrifteine Anzahl Filmhalter bekannt, von denen jeder einzelne einem speziellen Zweck dient. Dementsprechend ist für verschiedene Röntgenaufnahmen eine Anzahl separater Filmhalter notwendig. Dies führt zu einer größeren Lagerhaltung der Filmhalter und zu einer Belastung der Hilfspersonen des Arztes.

Es ist sehr wichtig, Röntgenuntersuchungen auf sichere und für den Patienten bequeme Weise durchzuführen und daß alle Arten der Untersuchungen rasch und unter Verwendung einer einzigen Einrichtung durchführbar sind.

Besondere Filmhalter zum sicheren Halten des Films während des tatsächlichen Belichtungsvorgangs sind bekannt. Die Erfordernisse für solche Filmhalter können wie folgt zusammengefaßt werden:

Der Filmhalter sollte hygienisch sein, d.h. entweder ein Einmalartikel sein oder sterilisiert werden können, er muß also keimfrei haltbar sein. Ferner muß der Filmhalter so haltbar sein, daß er alle Belichtungen übersteht, die an einem Patienten während einer Untersuchung vorgenommen werden.

Der Filmhalter soll vielseitig anwendbar sein und dementsprechend in den meisten oder vorzugsweise allen Situationen zur Anwendung kommen können, und zwar sowohl bei der Anfertigung von Periapikalaufnahmen wie auch bei der Erstellung von Bißflügelaufnahmen (Bite-Wing-Aufnahmen), und es sollte möglich sein, den Film sowohl hochkant als auch in waagrechter Anordnung zu verwenden. Bei Erstellung von Periapikalaufnahmen muß er so dick sein, daß die Filmkante oberhalb der Occlusionsebene liegt.

Andererseits muß er für die Herstellung von Bißflügelaufnahmen so dünn sein, daß die Zähne des Ober- und Unterkiefers nicht unnötig weit voneinander auf Abstand gehalten werden. Er muß mit einer Bißplatte von einer solchen Länge versehen werden, daß ein stabiler Zahnbiß bei den Fällen erhalten wird, bei denen der Film relativ weit von den Zähnen weggehalten wird, d.h. man verwendet für die Untersuchung eine Paralleltechnik wenn die Zähne des Oberkiefers untersucht werden oder die Front des Unterkiefers.

Eine kurze Bißplatte ist nötig, damit nicht z.B. das Kinn bei der Untersuchung der Seitenpartien des Unterkiefers im Wege steht.

Das Hilfsmittel muß leicht zu handhaben und auf einfache Weise von dem Zahnarzt oder der Helferin angewendet werden können. Es sollte für den Patienten kodfortabel sein, gleichzeitig aber einen stabilen Biß erlauben, so daß der Film in einer definierten Lage gehalten wird. Der Film soll gehalten werden, wobei eine Verbiegung des Films zu vermeiden ist. Es dürfen keine Schatten durch hohe Röntgenstrahlenabsorption auftreten.

Ferner ist es erwünscht, daß die Röntgenuntersuchungen rasch und effektiv durchführbar sind, so daß die Notwendigkeit für Wiederholungen auf ein Minimum reduziert werden kann.

Die bisher entwickelten Filmhalter haben die vorstehenden Erfordernisse und Aufgabenstellungen nur teilweise erfüllt.

Es ist Aufgabe der Erfindung, ein Hilfsmittel bzw. eine Vorrichtung zu schaffen, die die o.g. Erfordernisse und Wünsche bei niedrigem Kosteneinsatz erfüllt.

Die Aufgabe wird durch die in den Ansprüchen aufgeführten Merkmale gelöst.

Die Erfindung wird an einem Ausführungsbeispiel anhand von Zeichnungen erläutert. Es zeigen

Fig. 1 eine perspektivische Ansicht des Aufbaus der erfindungsgemäßen Vorrichtung,

Fig. 2 einen Schnitt durch einen Filmhalter als Teil der Vorrichtung,

Fig. 3 einen Führungsard als Teil der Vorrichtung,

Fig. 4 eine perspektivische Ansicht der Verbindung zwischen dem Filmhalter und dem Führungsarm,

Fig. 5 eine etwas abgewandelte Verbindung zwischen dem Filmhalter und dem Führungsarm,

Fig. 6 eine mit dem Führungsarm verbindbare Abschirmscheibe,

Fig. 7 einen Schnitt durch eine Filmhalterverlängerung,

Fig. 8 das Prinzip für die Durchführung einer sogenannten Bißflügelaufnahme,

Fig. 9 das Prinzip für eine Prämolaraufnahme am Unterkiefer,

Fig. 10 das Prinzip für die Aufnahme einer Molarregion des Oberkiefers,

Fig. 11 das Prinzip für die Durchführung einer Aufnahme des vorderen Bereichs des Unterkiefers.

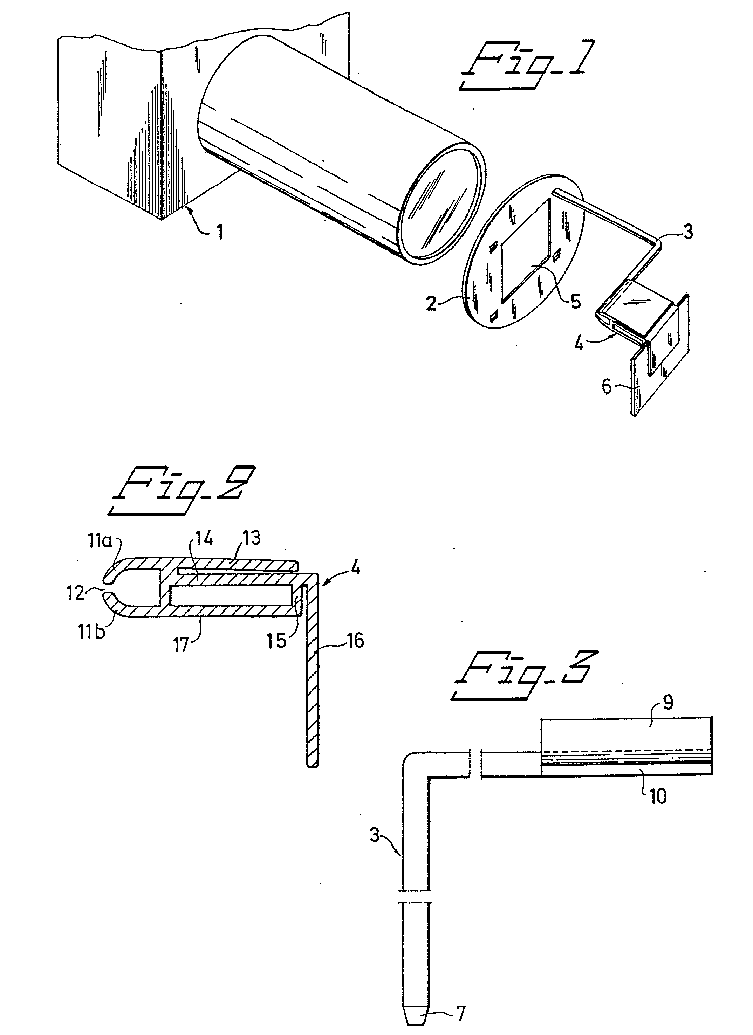

Die Fig. 1 zeigt im Prinzip die Durchführung einer erfindungsgemäßen Röntgenuntersuchung. Es ist bekannt, ein herkömmliches Röntgengerät 1 universell einstellbar zu lagern, so daß der Bildrahmen mit dem im Filmhalter positionierten, zu belichtenden Film zur Deckung gebracht werden kann.

Eine Abschirmscheibe 2 steht über einen Arm 3 in Verbindung mit einem Filmhalter 4. Die Verbindung zwischen der Abschirmscheibe 2 und dem Filmhalter 4 ist so gestaltet, daß eine Belichtungsöffnung 5 in der Abschirmscheibe immer mit einem Film 6 (Fig. 1) fluchtet, der in den Filmhalter eingesetzt ist, und es ist leicht verständlich, daß die Justage des Röntgengeräts beachtlich einfacher ist, weil der Zahnarzt oder die Helferin nur das Röntgengerät in Bezug auf die räumliche Lage der Abschirmscheibe 2 und hinsichtlich der Länge des Arms 3 einrichten muß, wobei die Abschirmscheibe immer in unmittelbarer Nähe des zylindrischen Endteils des Röntgengeräts, also des Senders, angeordnet wird. Dementsprechend hat es der Patient selbst in der Hand, das Führungsmittel, nämlich die Abschirmscheibe 2, in der korrekten Lage zu halten.

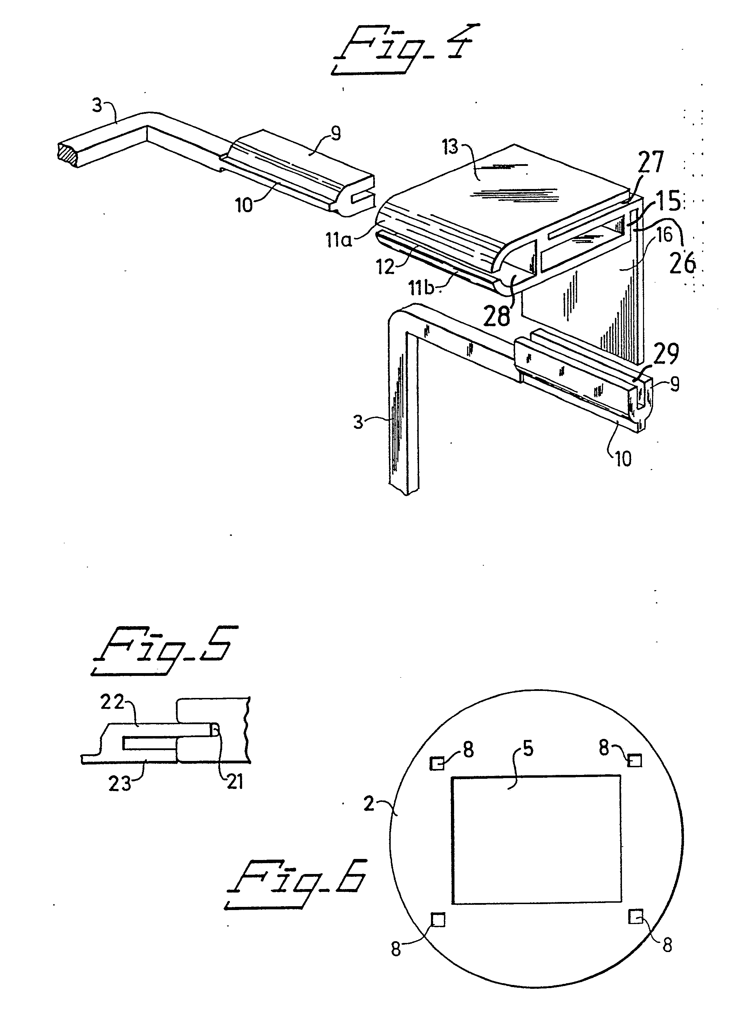

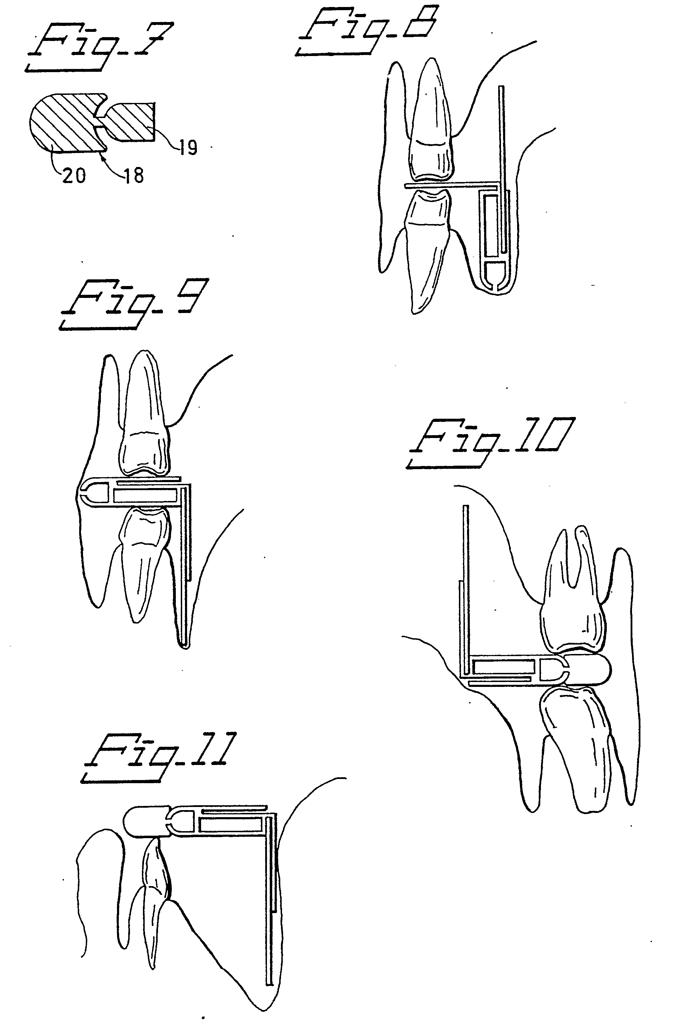

Zum Zweck der Ermöglichung des Einrichtens von hochkantstehendem bzw. liegendem Film und für die Untersuchungen, die weiter unten beschrieben werden, sind die Abschirmscheibe 2 und der Arm so aufgebaut, daß sie auf verschiedene Arten miteinander verbunden werden können. Zu diesem Zweck weist der Arm 3 (Fig. 3) ein konisches Verbindungsteil 7 auf, das im Querschnitt quadratisch ist. Die Abschirmscheibe 2 weist ferner eine Anzahl Öffnungen 8 (Fig. 6) auf, beim Ausführungsbeispiel sind dies vier Öffnungen, die quadratisch und konisch gestaltet sind, so daß das Verbindungsteil des Arms, durch Hineindrücken in die betreffende Öffnung zu einem Reibungsschluß zwischen dem Arm und der Abschirmscheibe führt. Wahlweise kann der Reibungsschluß auch durch eine Schnappanordnung oder eine andere Form einer rasch lösbaren Verbindung ersetzt werden. Durch diese Form der Anordnung und Konstruktion ist es möglich, daß der Arm in verschiedenen Lagen in Bezug auf die Abschirmscheibe zu dieser angeordnet und mit dieser in Verbindung gebracht werden kann.

Zur Verbindung des Arms mit dem Filmhalter in zwei verschiedenen Lagen weist der Arm 3 an seinem dem Verbindungsteil 7 abgekehrten Ende (Fig. 4) ein zweites Verbindungsteil mit einem Teil 9 U-förmigen Querschnitts auf, wobei die Schenkel des Teils 9 parallel zu einem weiteren Teil 10 angeordnet sind. Der Filmhalter 4 weist entsprechend der Darstellungen in Fig. 2 und 5 eine Fassung auf, die aus Backen 11a, 11b sowie einem Schlitz 12 besteht, die Fassung ist an die Teile 9, 10 angepaßt, die Teile 9, 10 passen gewissermaßen als Stecker in die Fassung 11a, 11b, 12. Der Arm 3 kann in den Filmhalter dadurch eingesteckt werden, daß die Teile 9, 10 des Arms in die Fassung 11a, 11b, 12 im Filmhalter eingeschoben werden. Wie aus Fig. 2 hervorgeht, weist der Filmhalter 4 zwei Wände 13, 14 auf, zwischen denen der Röntgenfilm angeordnet werden kann, und zwei Wände 15, 16 sind demgegenüber im rechten Winkel angeordnet, ein Röntgenfilm kann zwischen die beiden letzteren auch eingeschoben werden. Die Stärke der Wand 16 wird so gewählt, daß sich eine Anpassung an den Abstand ergibt, der zwischen den U-förmigen Schenkeln des Teils 9 besteht. Hierdurch kann der Arm mit der dünnen Wand 16 des Filmhalters in einer Weise verbunden werden, wie sie im rechten Bereich der Fig. 4 der Zeichnung zu sehen ist.

Bei manchen Untersuchungen ist eine größere Bißfläche bzw. Haltefläche notwendig als die, die sich durch die Wände 13 und 17 ergibt, und zu diesem Zweck kommt ein Verlängerungsteil 18 entsprechend Fig. 7 zur Anwendung. Dieses Verlängerungsteil weist ein Teil 19 auf, das in die Fassung paßt, die durch die Backen 11a und 11b sowie den Schlitz 12 gebildet ist, und ein Teil 20 bildet die eigentliche Verlängerung.

Aus den Fig. 10 und 11 geht ein Ausführungsbeispiel für Untersuchungen hervor, bei denen ein Verlängerungsteil nach Fig. 7 zur Anwendung kommt.

Fig. 8 zeigt die Anordnung für die Herstellung einer Bißflügelaufnahme (Bite-Wing-Bild), in diesem Fall kommt der Biß dadurch zustande, daß die dünne Wand 16 des Filmhalters festgehalten wird. Bei einer Untersuchung gemäß Fig. 9 erfolgt das Halten durch den Biß statt dessen dadurch, daß der dickere Teil des Filmhalters gehalten wird, der Film befindet sich in seiner liegenden, also querformatigen Anordnung. Auch bei einer Untersuchung nach Fig. 10 wird ein Film in liegender Anordnung gehalten, wobei auch das Verlängerungsteil zur Anwendung kommt. Schließlich wird auch bei der Untersuchung gemäß Fig. 11 das Verlängerungsteil angewandt, der Film steht aber aufrecht.

Wie aus der obenstehenden Beschreibung hervorgeht, ermöglicht das erfindungsgedäße Hilfsmittel die Durchführung von Röntgenuntersuchungen der meisten gängigen Arten durch Verwendung sehr einfacher und leicht zu handhabender Elemente, wobei gleichzeitig die Einstellung des Röntgengeräts auf einfache Weise möglich ist und das Risiko für eine unkorrekte Einstellung u.s.w., die eine Wiederholung der Aufnahme notwendig machen würde, drastisch reduziert wird.

Bei einem Ausführungsbeispiel des Verbindungsteils gemäß Fig. 5 ist der Filmhalter mit einem Schlitz 21 in seinem dickeren Bereich versehen, und der Verbindungsteil des Arms besteht aus zwei herausragenden Wänden 22, 23, von denen die Wand 22 länger als die Wand 23 ist. Der Abstand zwischen den Wänden 22, 23 ist so gewählt, daß er an die Wanddicke der dünneren Wand 16 des Filmhalters angepaßt ist, so daß sich ein Reibschluß hinsichtlich der beiden möglichen Verbindungen ergibt.

## Patentansprüche

1. Hilfsmittel für die Durchführung von Röntgenuntersuchungen an Zähnen, mit einem Filmhalter L-förmigen Querschnitts zur Aufnahme eines Röntgenfilms in zwei alternativen Ebenen, dadurch gekennzeichnet, daß der eine Schenkel (13) des Filmhalters (4) hohl ausgebildet ist, während der andere Schenkel eine Wand (16) hat, die zusammen mit einer Wand (15), des hohlen Schenkels (13) eine erste Aufnahmetasche (26) zur Halterung eines Teils eines Röntgenfilms (6) bildet, und daß Wände des hohlen Schenkels (13) teils eine zweite Aufnahmetasche (27) zur Halterung eines Teils eines senkrecht zum Röntgenfilm in der ersten Aufnahmetasche (26) ausgerichteten Röntgenfilms bilden und eine profilierte Ausnehmung (28) am freien Ende des hohlen Schenkels (13) bilden, und daß ein Verlängerungsorgan (3, 18) am freien Ende des hohlen Schenkels (13) eine zu der Ausnehmung (28) komplementär ausgeformte, aus Fassungsteilen (9, 10, 19) gebildete Fassung aufweist, die mit der Ausnehmung (28) des hohlen Schenkels (73) derart zusammenwirkt, daß das Verlängerungsorgan (3, 18) mit dem Filmhalter (4) lösbar verbindbar ist.

2. Hilfsmittel nach Anspruch 1, dadurch gekennzeichnet, daß die Ausnehmung (28) des hohlen Schenkels (13) eine längliche, verjüngte Austrittsöffnung (72) hat, die zentral am freien Ende des hohlen Schenkels (13) angeordnet ist, so daß die aus den Fassungsteilen (9, 10, 19) gebildete Fassung des Verlängerungsorgans (3, 18) seitlich und von je einer Seite aus in die Ausnehmung (28) einführbar ist dabei eine Entfernung des Verlängerungsorgans (3, 18) in Längsrichtung verhindert.

3. Hilfsmittel nach Anspruch 1, dadurch gekennzeichnet, daß die Ausnehmung (28) des einen Schenkels (13) des Filmhalters (4) im wesentlichen schwalbenschwanzförmige Gestalt aufweist, und daß auch die Fassung des Verlängerungsorgans (3) im wesentlichen schwalbenschwanzförmige Gestalt hat, jedoch auch mit einer Ausnehmung (29) von derartigen Abmessungen versehen ist, daß die Fassung des Verlängerungsorgans (3) das freie Ende des anderen Schenkels des Filmhalters (4) umgreifen kann.

4. Hilfsmittel nach Anspruch 1, dadurch gekennzeichnet, daß das Ende des hohlen Schenkels (13) gewölbt ist, und daß das Verlängerungsorgan (18) eine entsprechende, konkave Fläche und die gleiche nicke wie der hohle Schenkel (13) aufweist, so daß das Verlängerungsorgan (18) im Gebrauch eine Verlängerung des hohlen Schenkels (13) in dessen Längsrichtung darstellt.

5. Hilfsmittel nach Anspruch 1, dadurch gekennzeichnet, daß das Verlängerungsorgan (2) aus einem L-förmigen Arm besteht, der mit einer vorzugsweise kreisförmigen Abschirmscheibe (2) verbindbar ist.

6. Hilfsmittel nach Anspruch 5, dadurch gekennzeichnet, daß die Abschirmscheibe (2) eine vorzugsweise rechteckige, den Abmessungen des Bildbereichs entsprechende Belichtungsöffnung (5) aufweist.

7. Hilfsmittel nach einem der Ansprüche 5 oder 6, dadurch gekennzeichnet, daß die Abschirmscheibe (2) eine Anzahl vorzugsweise konisch zulaufender, und in ihrem Querschnitt rechteckiger Öffnungen (8) aufweist.

8. Hilfsmittel nach Anspruch 7, dadurch gekennzeichnet, daß die Öffnungen (8) mit einem entsprechenden, vorzugsweise konischen Verbindungsteil (7) des Verlängerungsorgans (3) zusammenarbeiten und so angeordnet sind, daß die Belichtungsöffnung (5) mit dem Film (6) unabhängig von dessen Anordnung in aufrechter nach rechts, links, oben oder unten weisender Lage fluchtet.

9. Hilfsmittel nach Anspruch 1, dadurch gekennzeichnet, daß der hohle Schenkel (13) des Filmhalters (4) mit einem Schlitz (21) versehen ist, und daß der der Verbindung dienende Teil des Verlängerungsorgans (3) eine hervorstehende Wand (22) von einer solchen Stärke aufweist, daß sie in den Schlitz (21) paßt und dort durch Reibschluß gehalten wird, und daß eine zurückstehende Wand (23) auf Abstand zu der hervorstehenden Wand (22) vorgesehen ist sowie daß der zwischen den hervor- bzw. zurückstehenden Wänden (22, 23) bestehende Abstand der Stärke der Wand (16) des anderen Schenkels des Filmhalters entspricht.

**Claims**

1. Aids for accomplishing tooth X-ray examinations having a film holder with an L-shaped cross-section for holding an X-ray film in two alternate planes, characterized in that one of the legs (13) of the film holder (4) is hollow while the other one has a wall (16), which along with a wall (15) of the hollow leg (13) is making a first female piece (26) for holding a part of the X-ray film (6), and that walls of the hollow legs (13) are partly making a second female piece (27) for holding a part of an X-ray film oriented perpendicular to the X-ray film in the first female piece (26) and make a shaped opening (28) at the free end of the hollow leg (13), and that an extension leg (3, 18) at the free end of the hollow leg (13) has a socket made from socket parts (9, 10, 19) designed complementary compared with the opening (28), which socket co-operates with the opening (28) of the hollow leg (13) in that way, that the extension leg (3, 18) is detachably connectable with the film holder (4).

2. Aids of claim 1, characterized in that the opening (28) of the hollow leg (13) has an elongated, smaller slot (12) being centrally located at the free end of the hollow leg (13) so that the socket of the extension leg (3, 18) made from the socket parts (9, 10, 19) may be inserted sidewise and from any of two sides into the opening (28) whereby preventing that the extension leg (3, 18) may be put off lengthwise.

3. Aids of claim 1, characterized in that the opening (28) of one of the legs (13) of the film holder (4) mainly has a dovetailed shape and that also the socket of the extension leg (3) has a dovetailed shape, but has also a female part (29) of such a size that the socket of the extension leg (3) is able to outline the free end of the other leg of the film holder.

4. Aids of claim 1, characterized in that the end of the hollow leg (13) is arched and that the extension leg (18) has a respective convexed surface and is in the same thickness as the hollow leg (13), so that the extension leg (18) when used is an extension of the hollow leg (13) in its longitudinal direction.

5. Aids of claim 1, characterized in that the extension leg (3) consists of an L-shaped arm connectable with an preferably circular shield disc (2).

6. Aids of claim 5, <u>characterized</u> in that the shiled disc (2) has a preferably rectangular exposure opening (5) in its size corresponding to the picture.

7. Aids of one of the claims 5 or 6, <u>characterized</u> in that the shield disc (2) has a number of preferably conically shaped openings (8) having a rectangular cross-section.

8. Aids of claim 7, <u>characterized</u> in that the openings (8) co-operate with a respective, preferably conically shaped connection part (7) of the extension leg (3) and are oriented in such that the exposure opening (5) aligns with the film (6) independent from its upright to the right, left, up or down directed orientation.

9. Aids of claim 7, <u>characterized</u> in that the hollow leg (13) of the film holder (4) is provided with a slot (21), and that the portion of the extension leg (3) used as a connection has a protruding wall (22) of such thickness, that it meets the slot (21) and is held therein by frictional force, and that there is provided a wall (23) in a distance behind the protruding wall (22) and that the distance between both of the walls (22, 23) corresponds to the thickness of the wall (16) of the other leg of the film holder.

**Revendications**

1. Accessoire pour l'exécution d'examens radiologiques sur des dents, comportant un porte-film à section en L destiné à recevoir un film radiographique dans deux plans possibles, caractérisé en ce que l'une des ailes (13) du porte-film (4) est de constitution creuse, tandis que l'autre aile présente une paroi (16) qui en même temps qu'une paroi (15) de l'aile creuse (13), forme un premier repli de logement (26) pour retenir une partie d'un film radiographique (6) et les parois de l'aile creuse (13) forment en partie un deuxième repli de logement (27) pour retenir un film radiographique aligné perpendiculairement au film radiographique qui se trouve dans le premier repli de logement (26) et forment un evidement profilé (28) à l'extrémité libre de l'aile creuse (13), et en ce qu'un organe de prolongement (3, 18) de l'extrémité libre de l'aile creuse (13) présente une monture façonnée de façon complémentaire de l'évidement (28), formée de parties de monture (9, 10, 19), qui coopère avec l'évidement (28) de l'aile creuse (13) de telle sorte que l'organe de prolongement (3, 18) est relié de façon détachable au porte-film (4).

2. Accessoire selon la revendication 1, caractérisé en ce que l'évidement (28) de l'aile creuse (13) présente une ouverture de sortie allongée rétrécie (12) qui est disposée au centre de l'extrémité libre de l'aile creuse (13), de sorte que la monture de l'organe de prolongement (3, 18), formée des parties de monture (9, 10, 19) peut être introduite latéralement et chaque fois par un côté dans l'evidement (28) et empêche alors un retrait de l'organe de prolongement (3, 18) en direction longitudinale.

3. Accessoire selon la revendication 1, caractérisé en ce que l'évidement (28) de l'une des ailes (13) du porte-film (4) presente pratiquement une forme en queue d'aronde, et en ce que la monture de l'organe de prolongement (3) présente pratiquement une forme en queue d'aronde, mais est aussi munie d'un évidement (29) de dimensions telles que la monture de l'organe de prolongement (3) peut entourer l'extrémité libre de l'autre aile du porte-film (4).

4. Accessoire selon la revendication 1, caractérisé en ce que l'extrémité de l'aile creuse (13) est bombée, et en ce que l'organe de prolongement (18) présente une surface concave correspondante et a la même épaisseur que l'aile creuse (13), de sorte qu'en service, l'organe de prolongement (18) constitue un prolongement de l'aile creuse (13) dans la direction longitudinale de celle-ci.

5. Accessoire selon la revendication 1, caractérisé en ce que l'organe de prolongement (3) est forme d'un bras en L qui peut être relié à une plaque de blindage (2), de préférence circulaire.

6. Accessoire selon la revendication 5, caractérisé en ce que la plaque de blindage (2) présente une ouverture d'exposition (5), de préférence rectangulaire, correspondant aux dimensions de la région d'image.

7. Accessoire selon l'une des revendications 5 et 6, caractérisé en ce que la plaque de blindage (2) presente un certain nombre d'ouvertures (8) rétrécissant coniquement et rectangulaires dans leur section.

8. Accessoire selon la revendication 7, caractérisé en ce que les ouvertures (8) coopèrent avec une partie de liaison correspondante (7), de préférence conique de l'organe de prolongement (3) et sont disposées de telle sorte que l'ouverture d'exposition (5) s'aligne sur le film (6) indépendamment de la disposition de celuici en position dressée tournée vers la droite, vers la gauche, vers le haut ou vers le bas.

9. Accessoire selon la revendication 1, caractérisé en ce que l'aile creuse (13) du porte-film (4) est munie d'une fente (21), et en ce que la partie de l'organe de prolongement (3) qui sert à la liaison présente une paroi en saillie (22) d'une épaisseur telle qu'elle s'adapte dans la fente (21) et y est retenue par frottement, et en ce qu'une paroi en retrait (23) est prévue avec espacement par rapport à la paroi en saillie (22), et en ce que l'espacement existant entre les parois en saillie et en retrait (22, 23) correspond à l'épaisseur de la paroi (16) de l'autre aile du porte-film.

_Fig_1

1

2

5

3

4

6

_Fig_2

11a

14    13

12

11b

17    15

4

16

_Fig_3

9

10

3

7

*Fig.4*

*Fig.5*

*Fig.6*

Fig.7

Fig.8

Fig.9

Fig.10

Fig.11